# EUROPÄISCHE PATENTANMELDUNG

(11) **EP 1 103 301 A1**
(43) Veröffentlichungstag der Anmeldung: **30.05.2001**
(21) Anmeldenummer: 00123190.1
(22) Anmeldetag: 26.10.2000
(51) Int. Cl.: B01J 23/00, B01J 23/887, C07C 45/36, C07C 47/55

(54) **Verfahren zur katalytischen Herstellung von Halogenbenzaldehyden und Katalysatoren zur Durchführung des Verfahrens**

(30) Priorität: 29.11.1999 DE 19957416
(71) Anmelder: Aventis Research & Technologies GmbH & Co KG, 65926 Frankfurt am Main (DE)
(72) Erfinder: Zeyss, Sabine, Dr., 65779 Kelkheim-Fischbach (DE); Dingerdissen, Uwe, Dr., 64342 Seeheim-Jugenheim (DE); Riermeier, Thomas, Dr., 65439 Flörsheim (DE); Centi, Gabriele, Prof., 98158 Faro Superiore-Messina (IT)
(74) Vertreter: Ackermann, Joachim, Dr.

(57) **Zusammenfassung**

Die Erfindung betrifft ein Verfahren zur Herstellung von Halogenbenzaldehyden durch katalytische Gasphasenoxidation eines substituierten Toluols mit Sauerstoff sowie neuartige Katalysatoren der Formel (III) die bei der Herstellung von Halogenbenzaldehyden eingesetzt werden können.

Me¹ ₐMe² _{b}Me³ _{c}Oₓ (III)

worin
- Me¹: für mindestens eines der Elemente aus der Gruppe Lithium, Natrium, Kalium, Rubidium, Cäsium, Antimon, Cer oder Wismut
- Me²: für mindestens eines der Elemente aus der Gruppe Eisen, Cobalt, Nickel, Zinn, Wolfram, Rhenium, Tantal, Palladium, Phosphor, Chrom, Mangan, Kupfer, Zink, Silber, Bor, Samarium, Barium, Calcium Magnesium Tellur oder Selen und
- Me³: für mindestens eines der Elemente aus der Gruppe Vanadium, Molybdän oder Niob steht
und das Atomverhältnis der jeweiligen Elemente bezogen auf c = 1 unabhängig voneinander für a zwischen 0 und 0,6 und für b zwischen 0 und 0,7 liegt, wobei das Verhältnis (a+b)/c zwischen 0,2 und 0,9 liegt und freie positive Valenzen mit x Sauerstoffatomen abgesättigt sind.

## Beschreibung

Die Erfindung betrifft ein Verfahren zur Herstellung von Halogenbenzaldehyden durch katalytische Gasphasenoxidation eines substituierten Toluols mit Sauerstoff sowie neuartige Katalysatoren die bei der Herstellung von Halogenbenzaldehyden eingesetzt werden können.

Zur Herstellung von Halogenbenzaldehyden durch Gasphasenoxidation von Toluol mit molekularem Sauerstoff sind als Katalysatoren Silbervanadat entweder allein oder in Kombination mit Blei- und Eisenoxid (SU 495 301, DE 1 295 538, NL 7 607 598) sowie ein Mischoxidkatalysator bestehend aus Kupfer-, Eisen-, Uran-, Blei-, Tellur-, Molybdän-, und Phosphoroxid (US 4 390 728) bekannt. Mit diesem Katalysator wird bei 38 % Umsatz Benzaldehyd mit 64 % Selektivität gebildet.

Höhere Ausbeuten werden bei der Oxidation von methoxy-, phenoxy-, oder tert.butylsubstituierten Toluolen erhalten, was auf den positiven Induktionseffekt dieser Gruppen zurückgeführt wurde (Grabowska, 1987; Ueshima, 1992; Constantini, 1986). So können p-Methoxytoluol und p-Phenoxytoluol an einem Vanadium-Thallium-Cäsium-Antimon- bzw. Vanadium-Cäsium-Kalium-Phosphor-Eisen-Cobalt-Katalysator bei 450°C mit über 75 % Ausbeute zu den entsprechenden Aldehyden oxidiert werden (EP 226 640). Am letztgenannten Katalysator wird entsprechend dem geringen elektronenziehenden Effekt des tert.-Butylsubstituenten im Vergleich zum Methoxy- und Phenoxysubstituenten p-tert.-Butylbenzaldehyd mit 56 % Ausbeute erhalten (EP 226 640).

Im Gegensatz zu Toluolen mit elektronenschiebenden Substituenten am aromatischen Ring wurden bis dahin nur geringe Ausbeuten im Falle der Gasphasenoxidation von Halogentoluolen erhalten, was das Vorurteil zu bestätigen schien, daß hohe Selektivitäten im Falle von elektronenziehenden Substituenten nicht zu erreichen sind. Nach DE 1 202 774 kann 2,6-Dichlortoluol lediglich mit 13 % Ausbeute zum entsprechend substituierten Benzaldehyd an Wismutmolybdat oxidiert werden. Der Umsatz bei einmaligem Reaktordurchgang beträgt nur 20 %, obgleich die Reaktionstemperatur bei 580°C liegt. Bei der Oxidation von p-Chlortoluol in einer Natrium/Kalium-Schmelze beträgt die Ausbeute sogar nur 4 % (US 4 885 412).

In EP 723 949 A1 ist ein Verfahren zur Herstellung von Halogenbenzaldehyden durch direkte Oxidation von entsprechend substituierten Toluolen beschrieben. Das Verfahren besitzt eine ausgezeichnete Selektivität und damit verbunden eine Steigerung der Ausbeute bezüglich der eingesetzten Menge an Edukt. Ein Nachteil des Verfahrens stellt allerdings das weiterhin niedrige Umsatzniveau dar.

Aufgabe der vorliegenden Erfindung ist es nun ein Verfahren zur Herstellung von Halogenbenzaldehyden zu entwickeln und einen geeigneten Katalysator dazu bereitzustellen, so daß die Reaktion von halogenierten Toluolen zu den entsprechenden Benzaldehyden bei gleichbleibender Selektivität aber erhöhtem Umsatz durchgeführt werden kann und damit eine erhöhte Ausbeute an Halogenbenzaldehyden bezogen auf das Edukt erreicht wird.

Die Aufgabe wird gelöst durch ein Verfahren zur Herstellung von Halogenbenzaldehyden der allgemeinen Formel (I) 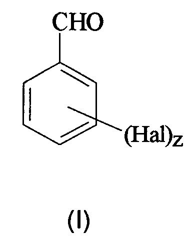 wobei Hal für Fluor, Chlor, Brom, Jod steht und z = 1, 2, 3 oder 4 ist durch katalytische Gasphasenoxidation aus den entsprechenden Halogentoluolen der allgemeinen Formel (II). 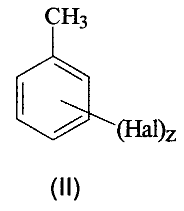 mit Sauerstoff in Gegenwart eines Katalysators der Formel (III)

Me¹ ₐMe² _{b}Me³ _{c}Oₓ (III)

worin
- Me¹: für mindestens eines der Elemente aus der Gruppe Lithium, Natrium, Kalium, Rubidium, Cäsium, Antimon, Cer oder Wismut
- Me²: für mindestens eines der Elemente aus der Gruppe Eisen, Cobalt, Nickel, Zinn, Wolfram, Rhenium, Tantal, Palladium, Phosphor, Chrom, Mangan, Kupfer, Zink, Silber, Bor, Samarium, Barium, Calcium Magnesium Tellur oder Selen und
- Me³: für mindestens eines der Elemente aus der Gruppe Vanadium, Molybdän oder Niob steht
und das Atomverhältnis der jeweiligen Elemente bezogen auf c = 1 unabhängig voneinander für a zwischen 0 und 0,6 und für b zwischen 0 und 0,7 liegt. Das Verhältnis (a+b)/c liegt dabei zwischen 0,2 und 0,9. Die freien positiven Valenzen sind mit x Sauerstoffatomen abgesättigt.

Die selektive Oxidationsreaktion zu den Halogenbenzaldehyden kann in einem Wirbelschicht- oder Festbettreaktor erfolgen. Wird der Katalysator-Edukt-Kontakt in einer Wirbelschicht hergestellt sind Partikelgerößen des Katalysators zwischen 10µm und 200µm vorteilhaft, bevorzugt sind Partikelgrößen zwischen 50µm und 100µm.

Als Oxidationsmittel wird Sauerstoff oder ein sauerstoffhaltiges Gas wie z. B. Luft oder einer Mischung aus Sauerstoff und einem Inertgas wie Stickstoff, Kohlendioxid, Wasserdampf oder einem Edelgas eingesetzt. Der Gehalt an gasförmigem Halogentoluol in der Ausgangsmischung ist in weiten Bereichen variierbar, wobei die Selbstentzündungstemperatur bzw. die Explosionsgrenze die einsetzbare Eduktkonzentration nach oben begrenzen. Als vorteilhaft hat sich ein Eduktstrom von 0,5 bis 5,0 Vol.-% Halogentoluol und 99,5 bis 95,0 Vol.-% Luft erwiesen. Der Volumenstrom im Reaktor besitzt unter Standarddruck und -temperatur (10⁵ Pa, 273 K, (STP)) eine Raumgeschwindigkeit von 100 bis 30000 h⁻¹. Die Reaktionstemperatur liegt zwischen 300° und 600°C, bevorzugt zwischen 400° und 500°C.
Die Reaktion kann im "single-pass"-Verfahren betrieben werden, aber auch ein Mehrfachdurchlauf des Reaktionsansatzes durch den Reaktor ist möglich.

Als Edukte sind die Fluor-, Chlor- und/oder Bromsubstituenten enthaltenden Toluole bevorzugt. Besonders bevorzugt sind deren monosubstituierten Derivate.

Ein weiterer Gegenstand der vorliegenden Erfindung ist der Katalysator selbst.

Der Katalysator kann in reiner Form vorliegen, mit einem Trägermaterial gemischt sein oder auf einem Trägermaterial fixiert sein. Beispiele für geeignete Trägermaterialien sind Aluminiumoxide, Keramiken, Kieselgur, Kieselgel, Siliziumcarbid oder -oxid, geglühtes Siliziumoxid oder Titan- oder Zirkoniumoxid.

Zur Herstellung des Katalysators können neben den entsprechenden Oxiden der einzelnen Komponenten auch andere, bevorzugt wasserlösliche Substanzen eingesetzt werden, die leicht durch Erwärmung in ihre korrespondierenden Oxide umgesetzt werden können. Solche Substanzen sind z. B. Ammoniumsalze, Nitrate, Sulfate, Halogenide, Hydroxide und Salze von organischen Säuren der einzelnen Komponenten.

Zur Vermischung der Komponenten können beispielsweise wäßrige Lösungen der Salze hergestellt und miteinander vermischt werden. Ein etwaiger Träger kann ebenfalls in der Mischung suspendiert werden. Nach ausfallen, filtrieren und waschen des festen Zwischenproduktes wird der Feststoff bei Temperaturen zwischen 100° und 250°C getrocknet. Dann wird das Zwischenprodukt bei Temperaturen von 300° bis 1000°C, bevorzugt zwischen 400° und 800°C zum entsprechenden Katalysator calciniert. Die Calcinierung erfolgt durch Erwärmung des Feststoffes innerhalb von 2 bis 48 Stunden, wobei ein ansteigender Temperaturgradient von 20° bis 100°C pro Stunde verwendet wird. Der erhaltene pulverförmige Katalysator kann z. B. durch kneten, fakultativ unter Zusatz eines Trägermaterials, in die benötigte Form gebracht werden. Um die Partikelgröße einzustellen kann das Katalysatorpulver zusätzlich gemahlen und/oder gesiebt werden oder, um sphärische Partikel zu erhalten kann das Katalysatorpulver mit einem geeigneten Solvenz in einer Trockenkammer bei Temperaturen von 100° bis 300°C sprühgetrocknet werden.

Die Mengen der eingesetzten Salze orientiert sich an der gewünschten Katalysatorzusammensetzung innerhalb der vorgegebenen Grenzen gemäß Formel (III)

Me¹ ₐMe² _{b}Me³ _{c}Oₓ (III)

worin
- Me¹: für mindestens eines der Elemente aus der Gruppe Lithium, Natrium, Kalium, Rubidium, Cäsium, Antimon, Cer oder Wismut
- Me²: für mindestens eines der Elemente aus der Gruppe Eisen, Cobalt, Nickel, Zinn, Wolfram, Rhenium, Tantal, Palladium, Phosphor, Chrom, Mangan, Kupfer, Zink, Silber, Bor, Samarium, Barium, Calcium Magnesium Tellur oder Selenfür und
- Me³: für mindestens eines der Elemente aus der Gruppe Vanadium, Molybdän oder Niob steht
und das Atomverhältnis der jeweiligen Elemente bezogen auf c = 1 unabhängig voneinander a zwischen 0 und 0,6, bevorzugt zwischen 0,05 und 0,6, und b zwischen 0 und 0,7, bevorzugt zwischen 0,025 und 0,6, liegt. Das Verhältnis (a+b)/c liegt dabei zwischen 0,2 und 0,9, bevorzugt zwischen 0,25 und 0,7. Alle freien positiven Valenzen sind mit x Sauerstoffatomen abgesättigt.

In einer bevorzugten Ausführungsform ist Me³ Niob und besonders bevorzugt Molybdän, Me¹ enthält mindestens ein Element aus gewählt aus der Gruppe Lithium, Natrium Kalium, Rubidium, Cäsium, Antimon und Cer, insbesondere aus der Gruppe Cäsium und Cer. Me² enthält bevorzugt Eisen und/oder Cobalt und/oder Wolfram.

Als besonders geeignet haben sich Katalysatoren des Typs Ce_{0,05-0,50}Fe_{0,2-0,4}MoOₓ erwiesen.

### Beispiele

### Beispiel 1 :

In 200 ml destilliertem Wasser werden bei Raumtemperatur 12,0g (NH₄)₆Mo₇O₂₄ ^{.} 4H₂O gegeben. Nach vollständiger Auflösung des Molybdänsalzes wird die Lösung unter ständigem rühren auf 50°C erhitzt (Lösung A). Eine zweite Lösung (Lösung B) wird durch Zugabe von 5g FeCI₃ 6H20 und 2.96g Ce(NO₃)₃ · 6H₂O zu 130 ml destilliertem Wasser hergestellt. Lösung B wird unter ständigem rühren zu Lösung A zugetropft, die Temperatur wird dabei auf 50°C gehalten. Die resultierende Lösung wird nach vollständiger Vereinigung der Lösungen A und B bei 50°C weitere 30 Minuten gerührt und dann auf Raumtemperatur gekühlt. Zur Fällung des Katalysatorzwischenproduktes wird die Lösung 24 Stunden bei Raumtemperatur stehen gelassen, dann abfiltriert und mit destilliertem Wasser solange gewaschen bis die Waschlösung einen pH-Wert von ca. 6 aufweist. Der Feststoff wird dann 12 Stunden bei 120°C getrocknet und anschließend in Gegenwart von Luft bei hohen Temperaturen calciniert. Das Temperaturprogramm der Calcinierung beinhaltet eine Erwärmung von 50°C pro Stunde mit isothermen Phasen von jeweils 2 Stunden bei 100°, 200°, 300° und 420°C. Nach Abkühlung auf Raumtemperatur wird der Katalysator im Mörser homogenisiert und eine Pulverfraktion von 40 - 60 mesh ausgesiebt.

Zur selektiven Oxidation von Halogentoluolen werden 0,3g des Katalysators mit 0,3g Quarzglaskugeln (40 bis 60 mesh) vermischt und dann in einem Quarzglasröhrchen mit einem inneren Durchmesser von 0,5 cm gegeben. Der Reaktor wird in einen elektrisch aufheizbaren Kupferbehälter eingeführt und zur Überwachung der Reaktionstemperatur wird in das Katalysatorbett ein Thermostat eingebracht. Das dem Reaktor zugeführte die Edukte enthaltende Anfangsgemisch des Reaktionsgases umfaßt 0,5 Vol.-% 3-Fluortoluol (alternativ 2- bzw. 4-Chlortoluol), 5 Vol.-% Sauerstoff, 20 Vol.-% Stickstoff und 74,5 Vol.-% Helium. Die Zufuhr des Gases erfolgt mit einem Volumenstrom von 6L/h (STP) bei einer Reaktortemperatur von 450° bzw. 500°C. Das 3-Fluorbenzaldehyd enthaltende, aus dem Reaktor austretende Gas, wird zur Reinigung und Aufnahme des Produktes durch -10°C kaltes Aceton geleitet. Das entstandene Kohlenmonoxid und Kohlendioxid werden gaschromatographisch erfaßt, die organischen Produkte werden mittels GC-MS quantitativ bestimmt. Als interner Standard dient Tridecan. Die Ergebnisse sind in Tabelle 1 zusammengefaßt.

### Beispiel 2 :

Versuchsdurchführung gemäß Beispiel 1, mit dem Unterschied, daß Lösung B anstatt 2,96g 6.40g Cernitrat enthält. Die Ergebnisse sind in Tabelle 1 wiedergegeben.

### Beispiel 3 :

Versuchsdurchführung gemäß Beispiel 1, mit dem Unterschied, daß Lösung B anstatt 2,96g 1,48g Cernitrat enthält. Die Ergebnisse sind in Tabelle 1 wiedergegeben.

### Beispiel 4:

Versuchsdurchführung gemäß Beispiel 1, mit dem Unterschied, daß Lösung B anstatt Cernitrat 6,5g Wismutnitratpentahydrat enthält. Die Ergebnisse sind in Tabelle 1 wiedergegeben.

### Beispiel 5:

Versuchsdurchführung gemäß Beispiel 1, mit dem Unterschied, daß Lösung B anstatt Cernitrat 3,1g Antimontrichlorid enthält. Die Ergebnisse sind in Tabelle 1 wiedergegeben.

### Beispiel 6 :

Versuchsdurchführung gemäß Beispiel 1, mit dem Unterschied, daß Lösung B anstatt Cernitrat 1,6g Antimontrichlorid enthält. Die Ergebnisse sind in Tabelle 1 wiedergegeben.

### Beispiel 7 :

Versuchsdurchführung gemäß Beispiel 1, mit dem Unterschied, daß Lösung B anstatt Cernitrat 0,8g Antimontrichlorid enthält. Die Ergebnisse sind in Tabelle 1 wiedergegeben.

### Beispiele 8 bis 11 :

Versuchsdurchführung gemäß Beispiel 1, mit dem Unterschied, daß Lösung B anstatt 2,96g 11,90 g Cernitrat enthält. Die Ergebnisse sind in Tabelle 1 wiedergegeben.

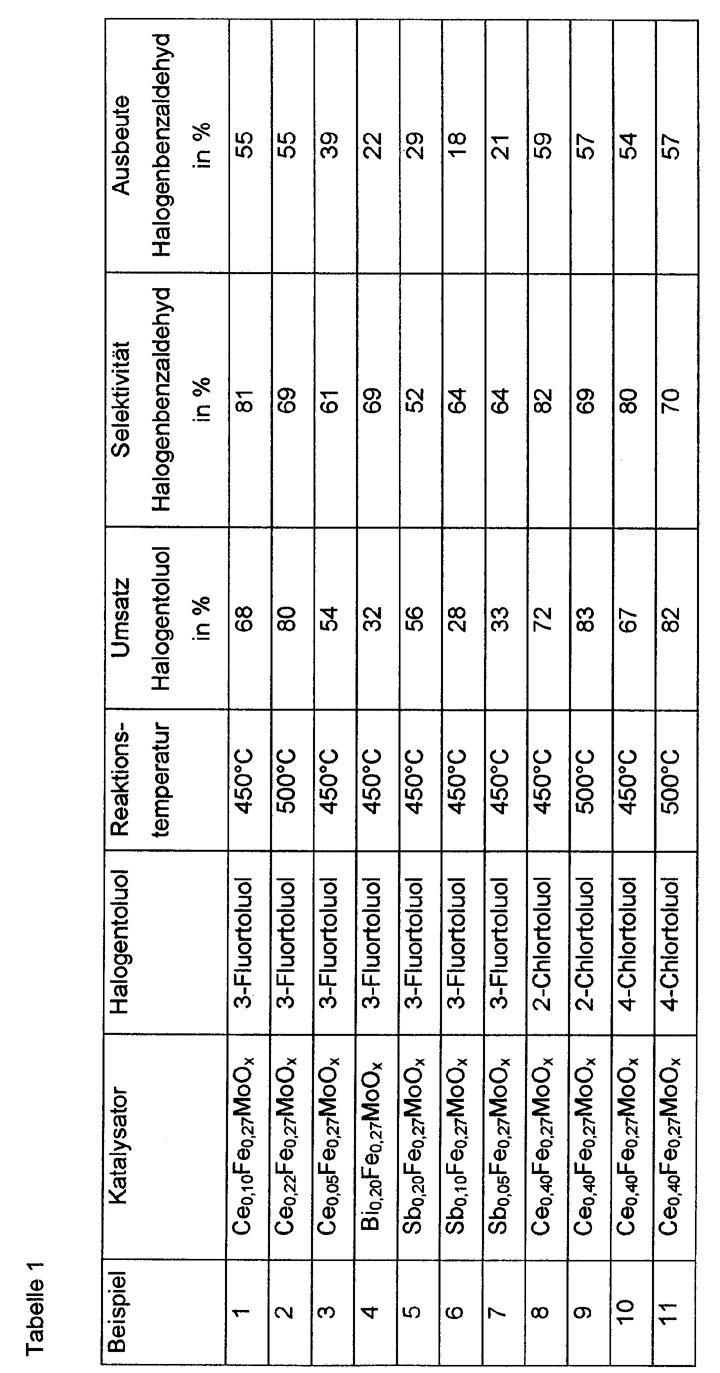

## Patentansprüche

1. Verfahren zur Herstellung von Halogenbenzaldehyden der allgemeinen Formel (I) 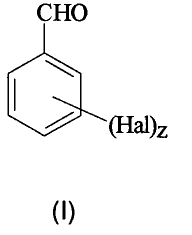 wobei Hal für Fluor, Chlor, Brom, Jod steht und z = 1, 2, 3 oder 4 ist durch katalytische Gasphasenoxidation aus den entsprechenden Halogentoluolen der allgemeinen Formel (II). 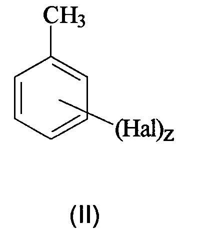 mit Sauerstoff dadurch gekennzeichnet, daß die Oxidation in Gegenwart eines Katalysators der Formel (III) durchgeführt wird,
Me¹ ₐMe² _{b}Me³ _{c}Oₓ (III)
worin
Me¹ für mindestens eines der Elemente aus der Gruppe Lithium, Natrium, Kalium, Rubidium, Cäsium, Antimon, Cer oder Wismut
Me² für mindestens eines der Elemente aus der Gruppe Eisen, Cobalt, Nickel, Zinn, Wolfram, Rhenium, Tantal, Palladium, Phosphor, Chrom, Mangan, Kupfer, Zink, Silber, Bor, Samarium, Barium, Calcium Magnesium Tellur oder Selen und
Me³ für mindestens eines der Elemente aus der Gruppe Vanadium, Molybdän oder Niob steht
und das Atomverhältnis der jeweiligen Elemente bezogen auf c = 1 unabhängig voneinander für a zwischen 0 und 0,6 und für b zwischen 0 und 0,7 liegt, wobei das Verhältnis (a+b)/c zwischen 0,2 und 0,9 liegt und freie positive Valenzen mit x Sauerstoffatomen abgesättigt sind.

2. Verfahren nach Anspruch 1 dadurch gekennzeichnet, daß die Oxidation in einem Wirbelschicht- oder in einem Festbettreaktor durchgeführt wird.

3. Verfahren nach Anspruch 1 oder 2 dadurch gekennzeichnet, daß als Oxidationsmittel als Oxidationsmittel ein Gasgemisch aus Sauerstoff und einem oder mehreren inerten Gasen, bevorzugt Stickstoff, Kohlendioxid, Argon oder besonders bevorzugt Luft eingesetzt wird.

4. Verfahren nach einem der vorherigen Ansprüche dadurch gekennzeichnet, daß die Reaktionstemperatur zwischen 300° und 600°C, bevorzugt zwischen 400° und 500°C liegt.

5. Verfahren nach einem der vorherigen Ansprüche dadurch gekennzeichnet, daß das Halogentoluol-Edukt in einer Konzentration von 0,5 Vol.-% bis 5,0 Vol.-% im Anfangsgasgemisch vorliegt.

6. Verfahren nach einem der vorherigen Ansprüche dadurch gekennzeichnet, daß das Gasgemisch den Reaktionsreaktor mit einer Raumgeschwindigkeit von 100 bis 30000 h⁻¹ (STP) durchströmt.

7. Katalysator der allgemeinen Formel (III)
Me¹ ₐMe² _{b}Me³ _{c}Oₓ (III)
worin
Me¹ für mindestens eines der Elemente aus der Gruppe Lithium, Natrium, Kalium, Rubidium, Cäsium, Antimon, Cer oder Wismut
Me² für mindestens eines der Elemente aus der Gruppe Eisen, Cobalt, Nickel, Zinn, Wolfram, Rhenium, Tantal, Palladium, Phosphor, Chrom, Mangan, Kupfer, Zink, Silber, Bor, Samarium, Barium, Calcium Magnesium Tellur oder Selenfür und
Me³ für mindestens eines der Elemente aus der Gruppe Vanadium, Molybdän oder Niob steht
und das Atomverhältnis der jeweiligen Elemente bezogen auf c = 1 unabhängig voneinander a zwischen 0 und 0,6 und b zwischen 0 und 0,7 liegt, wobei das Verhältnis (a+b)/c zwischen 0,2 und 0,9 liegt und alle freien positiven Valenzen mit x Sauerstoffatomen abgesättigt sind.

8. Katalysator nach Anspruch 7 dadurch gekennzeichnet, daß der Wert für a zwischen 0,05 und 0,6 und/oder der Wert für b zwischen 0,025 und 0,6 liegt.

9. Katalysator nach einem der Ansprüche 7 oder 8 dadurch gekennzeichnet, daß das Verhältnis (a+b)/c zwischen 0,25 und 0,7 liegt.

10. Katalysator nach einem der Ansprüche 7 bis 9 dadurch gekennzeichnet, daß
Me³ für Niob oder besonders bervorzugt für Molybdän steht und
Me¹ mindestens ein Element ausgewählt aus der Gruppe Lithium, Natrium Kalium, Rubidium, Antimon, Cäsium und Cer, insbesondere aus der Gruppe Cäsium und Cer enthält und/oder
Me² Eisen und/oder Cobalt und/oder Wolfram enthält.

11. Katalysator nach einem der Ansprüche 7 bis 10 dadurch gekennzeichnet, daß der Katalysator aus der Gruppe Ce_{0,05-0,50}Fe_{0,2-0,4}MoOₓ ist.

12. Katalysator nach einem der Ansprüche 7 bis 11 dadurch gekennzeichnet, daß der Katalysator in reiner Form vorliegt oder mit einem Trägermaterial vermischt ist oder auf einem Trägermaterial aufgebracht ist.

13. Katalysator nach Anspruch 12 dadurch gekennzeichnet, daß das Trägermaterial Aluminiumoxid, Keramik, Kieselgur, Kieselgel, Siliziumcarbid oder -oxid, geglühtes Siliziumoxid oder Titan- oder Zirkoniumoxid ist.

14. Verwendung eines Katalysators gemäß den Ansprüchen 7 bis 13 zur Herstellung von Halogenbenzaldehyden durch selektive Oxidation der korrespondierenden Halogentoluole.
